# EUROPEAN PATENT SPECIFICATION

(11) **EP 3 049 972 B1**
(45) Date of publication and mention of the grant of the patent: **06.11.2019**
(21) Application number: 14772367.0
(22) Date of filing: 25.09.2014
(51) Int. Cl.: G06F 3/0481, G16H 15/00

(54) **ENABLING REVIEW OF A MEDICAL IMAGE**
ERMÖGLICHEN DER ÜBERPRÜFUNG EINES MEDIZINISCHEN BILDES
ACTIVATION DE RÉVISION D'UNE IMAGE MÉDICALE

(30) Priority: 27.09.2013 EP 13186303
(43) Date of publication of application: 03.08.2016
(73) Proprietor: Koninklijke Philips N.V., 5656 AE Eindhoven (NL)
(72) Inventor: SERLIE, Iwo Willem Oscar, NL-5656 AE Eindhoven (NL)
(74) Representative: Zhu, Di
(86) International application number: PCT/EP2014/070471
(87) International publication number: WO 2015/044259

(56) References cited:
- EP-A1- 2 478 833
- US-A1- 2002 131 625
- US-A1- 2011 029 326
- US-A1- 2012 172 700

## Description

### FIELD OF THE INVENTION

The invention relates to a system and a method for enabling review of a medical image and for generating a report on the review of the medical image. The invention further relates to a workstation and an imaging apparatus comprising said system, and to a computer program product for causing a processor system to perform said method.

### BACKGROUND OF THE INVENTION

It is common in the medical field for users such as radiologists to review medical images and to document the review by generating a report. For example, a clinician may have a clinical query. In order to obtain an answer to such a clinical query, the clinician may issue a radiology request to the radiologist. In response, the radiologist may generate an imaging request for imaging a patient, review the resulting medical image(s), and generate a report comprising medical findings, conclusions, and other medical information. The radiologist or another medical user may be supported by a system in generating a report. Such a system may enable the user to generate the report by typing or dictating the medical information as free text, i.e., substantially unconstrained in content or structure.

It is known to obtain such reporting of medical information in a more standardized manner. For example, a publication titled "Medical Imaging on the Semantic Web: Annotation and Image Markup" by Daniel L. Rubin et al., AAAI Spring Symposium: Semantic Scientific Knowledge Integration, page 93-98, 2008, describes an image annotation tool which enables a user to provide a context for an annotation using a drop-down box. The image annotation tool uses the user-specified context in inferring the data fields that the user should collect for that annotation context. Accordingly, the image annotation tool requires users to record different types of information depending on the context.

It is also known to use macros to obtain reporting of medical information in a more standardized manner. In particular, such macros enable structured and/or codified descriptions to be generated of the medical information provided by the user. Here, the term 'structured' refers to the medical information being organized in accordance with standards or rules, such as those provided by radiology reporting guidelines. Moreover, the term 'codified' refers to the medical information being coded in accordance with similar or the same standards or rules to obtain the description. For example, the reporting guidelines may provide a standardized nomenclature which causes the medical information of "*organ* = *liver*" to be coded so as to provide a codified description being "*organ* = *RID56*".

The user may manually select such a macro. It is also known to suggest macros to the user based on the user starting to type free text and matching the free text to one or more macros. The macros may be suggested via auto-completion of the free text.

However, even if a system provides such macros to obtain a more standardized reporting of medical information, said standardized reporting still poses a significant burden to the user as the user has to manually provide the medical information, e.g., via the aforementioned typing in of the medical information as free text.

### SUMMARY OF THE INVENTION

It would be advantageous to provide a system or method which enables standardized reporting of medical information at a reduced burden to the user.

To better address this concern, a first aspect of the invention provides a system for enabling review of a medical image according to independent claim 1.

In a further aspect of the invention, a workstation and imaging apparatus is provided comprising the system set forth.

In a further aspect of the invention, a method is provided for enabling review of a medical image according to independent claim 14.

In a further aspect of the invention, a computer program product is provided comprising instructions for causing a processor system to perform the method set forth.

The aforementioned measures provide an image interface which obtains the medical image, e.g., from an internal or external storage medium. The medical image may be from a medical study constituted by a plurality of medical images. Moreover, a user interface subsystem is provided for interacting with a user. The user interface subsystem enables the user to interactively apply one or more graphical elements to the medical image during the review of the medical image. Here, the term 'apply to' refers to the graphical elements being placed on, overlaid over or otherwise attached to the medical image when the medical image is displayed to the user during the review. For example, the user interface subsystem may enable the user to select a graphical element by operating a mouse, and subsequently drag-and-drop the selected graphical element onto the medical image. Examples of such graphical elements include markers, annotations and parts of measurement tools. As a result, an application of one or more graphical elements to the medical image is obtained, as well as application data which represents the application of the one or more graphical element to the medical image. In other words, the application data expresses the characteristics of said application. For example, the application data may indicate which type of graphical element is applied to the medical image, its position relative to the medical image, etc.

Furthermore, a reporting subsystem is provided. The reporting subsystem is enabled to access a medical database which comprises medical information items. The medical information items represent computer-readable medical reference information such as text passages or schematic drawings from a medical textbook or medical guideline. Here, the term 'representing' refers to the medical reference information being directly expressed by the medical information item, e.g., by the medical information item comprising the medical reference information as a text passage or schematic drawing, or by such medical reference information being derivable from the medical information item, e.g., by decoding. The reporting subsystem retrieves a medical information item from the medical database. In selecting the medical information item amongst other medical information items, the reporting subsystem makes use of the application data which indicates the application of the one or more graphical elements to the medical image, as well as association data which enables the reporting subsystem to associate the application of the one or more graphical elements to at least one of the medical information items in the medical database. Finally, the reporting subsystem generates a report based on the retrieved medical information item.

The aforementioned measures have the effect that the system is enabled to generate a report without requiring the user to manually input medical information for the report. The present invention is at least in part based on the insight that the application of one or more graphical elements during the review of the medical image is indicative of what type of medical information the user is seeking to report during the review of the medical image. For example, if the user applies graphical elements from a lesion measurement tool to the medical image, it is likely that the user wishes to obtain and report medical information relating to the staging of such lesions. The present invention enables such medical information to be automatically obtained, namely based on association data linking the lesion measurement tool to relevant medical information items in the medical database.

The user therefore does not need manually input the medical information. Advantageously, the system reduces or entirely avoids the burden posed to the user when reporting on a review of a medical image. At the same time, by avoiding or reducing manual input of medical information by the user, the medical information is reported inherently in a standardized way, i.e., as determined by the contents of the association data and the medical information items in the medical database rather than at the hand of the user's discretion. Advantageously, a more structured reporting is obtained compared to free text input.

Optionally, the medical information item is associated with a codified version of the medical reference information, and the reporting subsystem is arranged for using the codified version of the medical reference information in generating the report.

The reporting subsystem is thus enabled to obtain a codified version of the medical reference information based on the medical information item. The codified version of the medical reference information adheres (more) to standards or rules, such as those provided by radiology reporting guidelines, than the medical reference information itself. For example, the medical reference information may be provided for the purpose of informing the user about a medical condition, whereas the codified version of the medical reference information may be specifically provided for the purpose of reporting on the medical condition. By using the codified version of the medical reference information to generate the report, a more standardized reporting on the medical image is obtained. Advantageously, a same standardized nomenclature and/or reporting structure may be obtained as if the system were to use a macro to generate a codified description of medical information provided by the user, yet without the user having to manually provide said medical information.

Optionally, the user interface subsystem is arranged for displaying the medical reference information as a selectable graphical element to the user, and the reporting subsystem is arranged for using the codified version of the medical reference information in generating the report based on the user selecting the selectable graphical element.

The medical reference information is thus presented onscreen as a graphical element such as a textbox, an inserted graphic or other type of overlay. The graphical element is a selectable graphical element in that the user may select the graphical element, e.g., by operating a mouse. The reporting subsystem is arranged for, upon selection of the graphical element by the user, using the codified version of the medical reference information in generating the report. Accordingly, the user is enabled to decide whether or not the report should be generated based on the retrieved medical information item, namely by selecting the graphical element or by refraining from said selecting. Such selecting of the graphical element signals to the system that the report should be generated based on the retrieved medical information item. Moreover, upon selecting the graphical element, the reporting subsystem uses the codified version of the selected medical reference information in generating the report. This aspect of the present invention is based on the insight that medical reference information is typically provided for the purpose of informing the user about a medical condition, whereas the codified version of the medical reference information is typically provided for the purpose of reporting on the medical condition. Accordingly, during it may be desirable to display the medical reference information to the user during the review, i.e., to enable easily determining whether the retrieved medical information item is applicable to the present case, while subsequently including its codified version in the report.

Optionally, the reporting subsystem is arranged for using a macro to generate the codified version of the medical reference information from the medical reference information. By using a macro, the codified version of the reference information can be generated dynamically, e.g., at a time when the codified version is needed. It is therefore not needed to pre-generate and store the codified version of the medical reference information. Advantageously, the use of a macro enables codifying other information together with the medical reference information. For example, the medical reference information may be supplemented with patient-specific information when generating the report.

Optionally, the medical information item comprises the macro or a reference to the macro. In order to codify different types of medical information items, different macros may be required. By including the macro or a reference thereto in the medical information item itself, the system can easily obtain the required macro for a particular medical information item. Advantageously, it is not needed to otherwise search for a macro.

Optionally, the medical information items are cross-referenced via hyperlinks, and the user interface subsystem is arranged for enabling the user to interactively browse to another medical information item by selecting a hyperlink in the selectable graphical element. The user is thus enabled to browse from an initially retrieved medical information item to cross-referenced, and thus typically related, medical information items. Accordingly, if the system inadvertently retrieves a medical information item which is of lesser relevance, the user can interactively browse to a more relevant medical information item. Accordingly, if the initially retrieved medical information item is considered as a suggestion from the system, the user is enabled to fine-tune the suggestion before signaling the system to use the fine-tuned medical information item in generating the report. Advantageously, the user is enabled to influence and improve the reporting by the system in an intuitive manner.

Optionally, the selectable graphical element is a selectable overlay, and the user interface subsystem is arranged for displaying the medical reference information in the form of text or graphics in the selectable overlay. The medical reference information is thus displayed in the form of an overlay over the medical image. Such display is intuitive since it adheres well to current user interface paradigms. Advantageously, the overlay may be used to enable the user to select specific parts of the medical reference information, such as a text passage or a part of a schematic drawing, which is to be used in generating the report.

Optionally, the one or more graphical elements provide a measurement tool for enabling the user to perform a measurement on the medical image using the user interface subsystem, and the association data is constituted at least in part by measurement data representing the measurement. The reporting subsystem thus uses the measurement data to identify the medical information item. This aspect of the invention is based on the insight that the outcome of a measurement on the medical image may more specifically indicate a current medical or pathological condition. Accordingly, the reporting subsystem is enabled to retrieve a more relevant medical information item, namely one which takes into account the current medical or pathological condition. For example, if the user applies graphical elements from a lesion measurement tool to the medical image, it is likely that the user wishes to obtain and report medical information relating to the staging of such lesions. The measurement of the lesion may then be used to retrieve a medical information item for the stage of the lesion in the medical image. For example, in the case the lesion exceeds 5cm in diameter, the retrieved medical information item may be for a so-termed T2 or T3a stage.

Optionally, the reporting subsystem is arranged for using the measurement data in generating the report. The reporting subsystem thus automatically uses the measurement data to generate the report, e.g., so as to include the measurement in the report, thereby avoiding the need for the user to manually report the measurement. Advantageously, in case the reporting subsystem uses a macro to generate the codified version of the medical reference information from the medical reference information, the macro may use the measurement data as input, thereby providing a codified description which takes into account the measurement. Advantageously, a more complete report is obtained.

Optionally, the association data is constituted at least in part by a plurality of rules each linking a respective one of the one or more graphical elements to a respective one of the medical information items. Such a set of rules is well suited to link the application of the one or more graphical elements to the medical information item.

Optionally, the application data represents one or more of the group of: a type of the one or more graphical elements, a position of the one or more graphical elements in the medical image, and an image structure marked by the one or more graphical elements. The type of graphical element may indicate which medical information items are of relevance and which are not. For example, a lesion measurement tool may indicate that medical information items relating to lesions are of relevance. Similarly, a position of the one or more graphical elements in the medical image and an image structure marked by the one or more graphical elements may indicate medical information items of relevance. Advantageously, the reporting subsystem is enabled to retrieve a more relevant medical information item.

Optionally, the one or more graphical elements comprise one or more of the group of: a marker, an annotation and a measurement tool.

It will be appreciated by those skilled in the art that two or more of the above-mentioned embodiments, implementations, and/or aspects of the invention may be combined in any way deemed useful.

Modifications and variations of the workstation, the imaging apparatus, the method, and/or the computer program product, which correspond to the described modifications and variations of the system, can be carried out by a person skilled in the art on the basis of the present description.

A person skilled in the art will appreciate that the method may be applied to multi-dimensional image data, e.g. to two-dimensional (2-D), three-dimensional (3-D) or four-dimensional (4-D) images. A dimension of the multi-dimensional image data may relate to time. For example, a 3-D image may comprise a time domain series of 2-D images.

The medical image may be acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

The invention is defined in the independent claims. Advantageous yet optional embodiments are defined in the dependent claims.

### BRIEF DESCRIPTION OF THE DRAWINGS

These and other aspects of the invention are apparent from and will be elucidated with reference to the embodiments described hereinafter. In the drawings,
Fig. 1 shows a system according to the present invention, which comprises a user interface subsystem for enabling a user to review a medical image, and a reporting subsystem for generating a report during or after the review of the medical image;
Fig. 2 shows a method according to the present invention;
Fig. 3 shows a computer program product for performing the method;
Fig. 4 schematically shows a medical information item representing medical reference information, with a codified version of the medical reference information being generated from the medical reference information using a macro;
Fig. 5a shows a user interface for enabling the user to apply graphical elements of a measurement tool to the medical image during the review of the medical image, thereby enabling the user to perform a measurement on the medical image;
Fig. 5b shows a first overlay being added to the user interface which shows medical reference information which is associated with the measurement, namely probable stages of a liver lesion as well as a diameter threshold associated with said stages;
Fig. 6a shows a second overlay being added which shows text passages from a medical textbook which describe medical characteristics of the liver lesion stages, with the user selecting one of the liver lesion stages from the first overlay;
Fig. 6b shows a third overlay being added which shows a codified version of the medical reference information which has been generated by the system by applying a macro to the medical reference information and the measurement; and
Fig. 7 shows a fourth overlay which shows a schematic drawing of the liver, with the user selecting an anatomical location in the liver from the fourth overlay.

It should be noted that items which have the same reference numbers in different Figures, have the same structural features and the same functions, or are the same signals. Where the function and/or structure of such an item has been explained, there is no necessity for repeated explanation thereof in the detailed description.

### DETAILED DESCRIPTION OF EMBODIMENTS

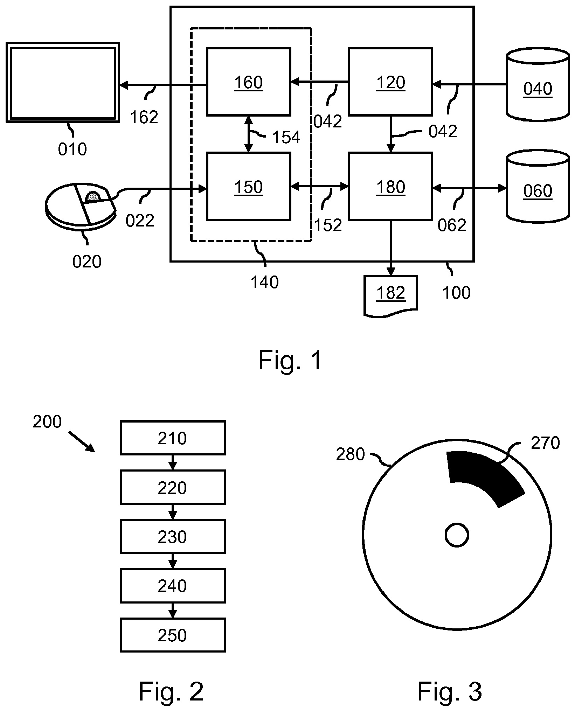

Fig. 1 shows a system 100 according to the present invention. The system 100 is arranged for enabling a user to review a medical image. The system 100 comprises an image interface 120 for obtaining the medical image 042. Fig. 1 shows the image interface 120 obtaining the medical image 042 from an external storage medium 040, as provided by, e.g., a Picture Archiving and Communication System (PACS). Alternatively, the image interface 120 may obtain the medical image 042 from an internal storage medium.

The system 100 further comprises a user interface subsystem 140. In general, the user interface subsystem 140 may enable the user to interact with the system 100 via a user interface established by the user interface subsystem 140 on a display 010. According to the present invention, the user interface subsystem 140 is arranged for enabling a user to apply one or more graphical elements to the medical image during the review of the medical image, thereby establishing application data representing application of the one or more graphical elements to the medical image. Fig. 1 shows the user interface subsystem 140 being comprised of a user input 150 and a display processor 160. However, this is not a limitation in that the user interface subsystem 140 may also take any other suitable form.

The user input 150 may be arranged for receiving user input data 022 from a user input device 020 such as a keyboard, mouse or touch sensitive surface. Moreover, the display processor 160 may be arranged for receiving the user input data 022 from the user input 150 via an internal exchange of messages 154. The display processor 160 may be arranged for presenting a user interface on the display 010. For that purpose, the display processor 160 may output display data 162 to the display 010. The user interface subsystem 140 may display the medical image 042 on the display 010, e.g., to enable the user to apply the one or more graphical elements to the medical image 042. For that purpose, the display processor 160 is shown to receive the medical image 042 from the image interface 120.

The system 100 further comprises a reporting subsystem 180. The reporting subsystem 180 is arranged for accessing a medical database 060 comprising medical information items which each represent medical reference information. Fig. 1 shows the medical database 060 being an external database. Alternatively, the medical database 060 may be an internal database. The reporting subsystem 180 is further arranged for retrieving a medical information item 062 from the medical database based on the application data and association data, the association data linking the application of the one or more graphical elements to the medical information item. In the example of Fig. 1, the association data is internal data of the reporting subsystem 180, whereas the application data is received from the user interface subsystem 140 via an internal exchange of messages 152. The reporting subsystem 180 is further arranged for generating a report 182 based on the medical information item as retrieved from the medical database 060. Fig. 1 schematically shows the report 182, i.e., in the form of an icon, with the report 182 being output from the system 100. Alternatively, the report 182 may be stored internally, in the medical database 060, etc.

An operation of the system 100 may be briefly explained as follows. The image interface 120 obtains the medical image 042. The user interface subsystem 140 enables the user to apply the one or more graphical elements to the medical image 042 during the review of the medical image. The user interface subsystem 140 thereby establishes application data representing the application of the one or more graphical elements to the medical image. The reporting subsystem 180 accesses the medical database 060 which comprises the medical information items. The reporting subsystem 180 then retrieves the medical information item 062 from the medical database based on the application data and the association data. Finally, the reporting subsystem 180 generates the report 182 based on the retrieved medical information item 062. For example, the reporting subsystem 182 may directly include the medical information item 062 in the report 182.

Fig. 2 shows a method 200 for enabling review of a medical image. The method 200 may correspond to an operation of the system of Fig. 1. However, the method 200 may also be performed in separation of said system, e.g., using a different system.

The method 200 comprises, in a first step titled "OBTAINING MEDICAL IMAGE", obtaining 210 the medical image. The method 200 further comprises, in a second step titled "OBTAINING APPLICATION DATA", enabling 220 a user to apply one or more graphical elements to the medical image during the review of the medical image, thereby establishing application data representing application of the one or more graphical elements to the medical image. The method 200 further comprises, in a third step titled "ACCESSING MEDICAL DATABASE", accessing 230 a medical database comprising medical information items which each represent medical reference information. The method 200 further comprises, in a fourth step titled "RETRIEVING MEDICAL INFORMATION ITEM FROM MEDICAL DATABASE", retrieving 240 a medical information item from the medical database based on the application data and association data, the association data linking the application of the one or more graphical elements to the medical information item. The method 200 further comprises, in a fifth step titled "GENERATING REPORT BASED ON MEDICAL INFORMATION ITEM", generating 250 a report based on the medical information item. It is noted that the above steps may be performed in any suitable order. In particular, the third step of accessing 230 the medical database may be performed in parallel with the earlier steps, i.e., the first step 210 and the second step 220.

Fig. 3 shows a computer program product 270 comprising instructions for causing a processor system to perform the aforementioned method 200. The computer program product 270 may be comprised on a computer readable medium 280, for example in the form of as a series of machine readable physical marks and/or as a series of elements having different electrical, e.g., magnetic, or optical properties or values.

An operation of the system may be further explained as follows.

To select the medical information item, the reporting subsystem makes use of association data. The association data may be constituted at least in part by a plurality of rules each linking the application of a respective one of the one or more graphical elements to a respective one of the medical information items. For example, the one or more graphical elements may comprise one or more of the group of: a marker, an annotation and a measurement tool. The association data may then represent a type of the one or more graphical elements in that a first rule links application of a marker to a different medical information item than application of a measurement tool. The rules may also distinguish between different types of markers, annotations and measurement tools. Additionally or alternatively, the rules may take into account a position of the one or more graphical elements in the medical image. The position may be a relative position between the multiple graphical elements, e.g., representing a measurement in the medical image. The position may also be an absolute position in the medical image, e.g., representing an anatomical location in the medical image. Accordingly, the rules may link different measurements and/or different anatomical locations to different medical information items. Additionally or alternatively, the rules may take into account an image structure marked by the one or more graphical elements. The system may detect which type of image structure is marked, e.g., using a region of interest detection technique. Alternatively, the one or more graphical elements may be associated with the image structure. As such, the application of the one or more graphical elements may imply or suggest that a particular type of image structure is marked.

It will be appreciated that the association data may also take various other forms. For example, the association data may be constitutes by the application data itself. In particular, instead of directly linking a respective one of the one or more graphical elements to a respective one of the medical information items, the reporting subsystem may take various data into account which is available to the system, which together constitutes the association data and which indirectly rather than directly identifies the medical information item to be retrieved. For example, the reporting subsystem may take contextual information into account such as metadata of the medical image, a patient record of a patient, a computer readable version of the clinical query and similar types of information. Together with the application data representing the application of the one or more graphical elements, the reporting subsystem is enabled to determine which medical information item is to be retrieved from the medical database, e.g., using known per se reasoning techniques.

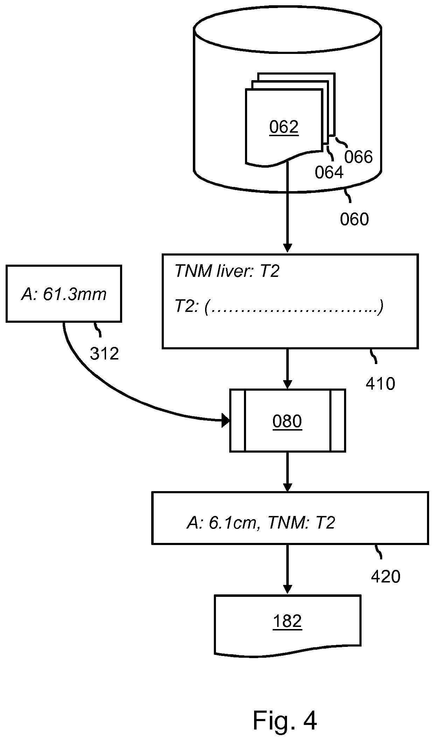

Fig. 4 schematically illustrates an optional aspect of the present invention, in that the medical information item 062 as retrieved by the reporting subsystem may be associated with a codified version of the medical reference information, and that the reporting subsystem may be arranged for using the codified version of the medical reference information in generating the report 182. Fig. 4 shows the medical database 060 comprising the medical information items 062-066, i.e., a plurality thereof. A first shown one of the medical information items 062-066, i.e., the retrieved medical information item 062, is shown to represent medical reference information 410 by a text box connected to the medical information item 062 comprising the text "*TNM liver: T2*". Here, the term TNM stands for Tumor, Nodes, Metastases and indicates a staging system for determining a stage of a lesion, whereas T2 corresponds to a particular stage in the staging system. Moreover, the medical reference information 410 may further comprise a textual explanation for criteria for the respective T2 stage, such as "*T2: Either a single tumor (any size) that has grown into blood vessels, OR more than one tumor where no tumor is larger than 5 cm (about 2 inches) across*". For clearness reasons, the above textual explanation has been omitted from Fig. 4. The medical information item 410 may have been obtained from a medical textbook which is accessible to the reporting subsystem via the medical database. In this example, the medical information item 410 may have been identified by the reporting subsystem based on the one or more graphical elements being part of a liver measurement tool, and the relative position of the graphical elements, as applied by the user, establishing a measurement of 61.3mm.

Fig. 4 further shows a codified version of the medical reference information 420 in the form of a text box comprising the text "*A: 6.1cm, TNM: T2*". The codified version of the medical reference information may provide a standardized nomenclature and/or reporting structure. For example, compared to the medical reference information 410, the stage is simply identified as "*TNM: T2*" instead of "*TNM liver: T2*" and the textual explanation provided by the medical reference information 410 is omitted. The codified version of the medical reference information 420 may be directly comprised in the medical information item 062 or be obtainable in a similar manner as the medical reference information 410, e.g., by decoding the medical information item 062. Alternatively, the reporting subsystem may be arranged for using a macro 080 to generate the codified version of the medical reference information 420 from the medical reference information 410. The use of macros to generate codified versions of medical information for the purpose of medical reporting is known per se from the field of medical reporting, and in particular, the field of radiology reporting. Such macros may provide a template for a user to fill in medical information, and subsequently codify the filled-in medical information to provide a codified version of the medical information. The reporting subsystem may use such a macro 080 to generate a codified version from the medical reference information 410, i.e., from the information provided by the medical information item 062 rather than medical information filled in by a user. As a result, the codified version of the medical reference information 420 may be included in the report 182 rather than the medical reference information 410 itself. The macro 080 may be comprised in the medical information item 062. Alternatively, the medical information item 062 may comprise a reference to the macro 080, thereby enabling the reporting subsystem to obtain the macro 080. Alternatively, the reporting subsystem may select the macro 080 from a plurality of macros based on the medical information item 062 and/or the application data, e.g., by analyzing the medical information item 062 and/or the application data to identify a most relevant macro.

Fig. 4 schematically illustrates a further optional aspect of the present invention, in that the macro 080 generates the codified version of the medical reference information 420 further based on measurement data 312. The measurement data 312 may have been obtained using the one or more graphical elements in that they may be part of a measurement tool, with their relative position providing a measurement in the medical image. For example, the user may have positioned the graphical elements within the medical image such that they measure a distance of 61.3mm. The macro 080 may codify the measurement, in that the measurement may be converted into a standardized nomenclature and/or reporting structure. As a result, in the example of Fig. 4, the measurement of 61.3mm results in the text *"A: 6.1cm"* being included in the report 182. It will be appreciated that the macro 080 may also codify various further information relevant to the review of the medical image.

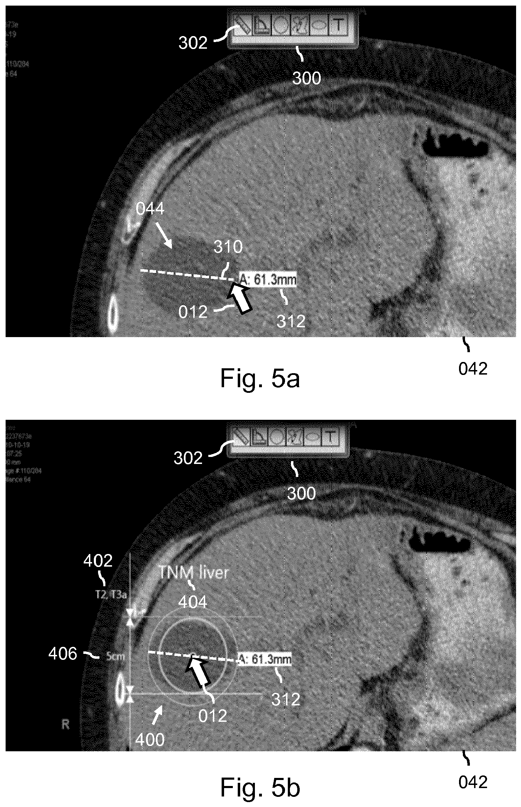

Figs. 5a - 7 illustrate an operation of the system by showing an example of a user interface as provided by the user interface subsystem to the user. In the following, the one or more graphical elements applied to the medical image by the user are those of a measurement tool. It will be appreciated, however, that the one or more graphical elements may also relate to, e.g., a marker or an annotation tool for generating an annotation.

Figs. 5a - 7 further illustrate optional aspects of the present invention, in that the user interface subsystem may be arranged for displaying the medical reference information as a selectable graphical element to the user, and that the reporting subsystem may be arranged for using the codified version of the medical reference information in generating the report based on the user selecting the selectable graphical element.

Fig. 5a shows a display output which presents the medical image 042 and the user interface to the user. The user interface comprises a menu bar 300 which is comprised of a plurality of icons representing respective image measurement and annotation tools. Said tools may be used by the user while reviewing the medical image 042. Upon selection, each tool may provide one or more graphical elements which may be applied to the medical image by the user. Fig. 5a shows the user having selected a left-most icon 302 of the plurality of icons, thereby selecting a measurement tool, i.e., a virtual ruler. To select said tool, the user may make use of an onscreen pointer 012 which may be controlled by the user by operating the user input device. As a result, the user is enabled to apply the virtual ruler 310 to the medical image 042. The virtual ruler 310 is represented by a graphical element in the form of a dashed line. Fig. 5a shows the user applying the virtual ruler 310 to measure a distance, namely of diameter of an image region 044 of a suspected lesion. The distance is indicated to the user as an overlay showing the measurement 312, namely "*A: 61.3mm*".

Fig. 5b shows a first overlay 400 being added to the user interface which shows medical reference information which is associated with the measurement, namely probable stages 402 of a liver lesion being *"T2, T3a*", a name 404 associated with the overlay being "*TNM liver*", and diameter threshold 406 associated with said stages being "*5cm*". In addition, the first overlay 400 provides visual indications of, amongst others, the diameter threshold, which are graphically represented as lines and circles. The medical reference information 402-406 may be retrieved by the reporting subsystem based on the type of the measurement tool, i.e., being a virtual ruler, as well as the measurement itself, i.e., 61.3mm. As such, the reporting subsystem is enabled to determine which staging information constitutes relevant medical reference information for such a measurement and accordingly retrieve a medical information item which provides said medical reference information. In particular, the measurement of 61.3mm may indicate to the reporting subsystem that the stages T2 and T3a are relevant stages and accordingly retrieve a medical information item providing medical reference information 402-406 for said stages. It is noted that in general, the data of such measurements may at least in part establish the association data used by the reporting subsystem to identify and retrieve the medical information item.

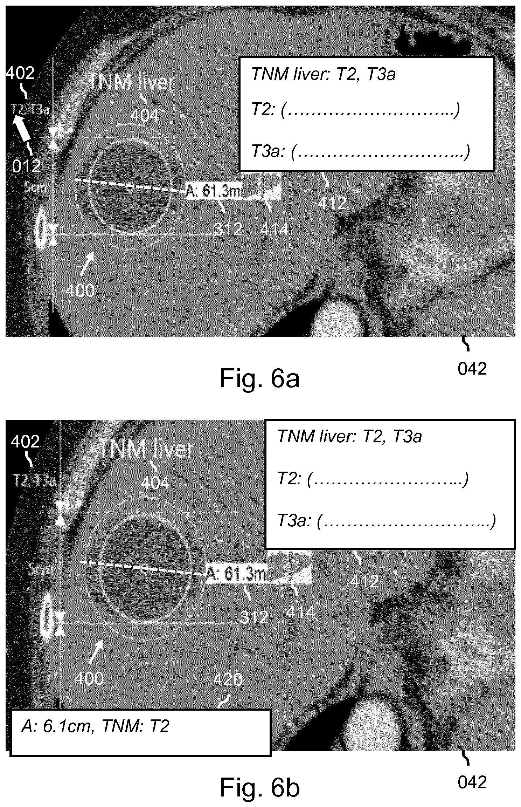

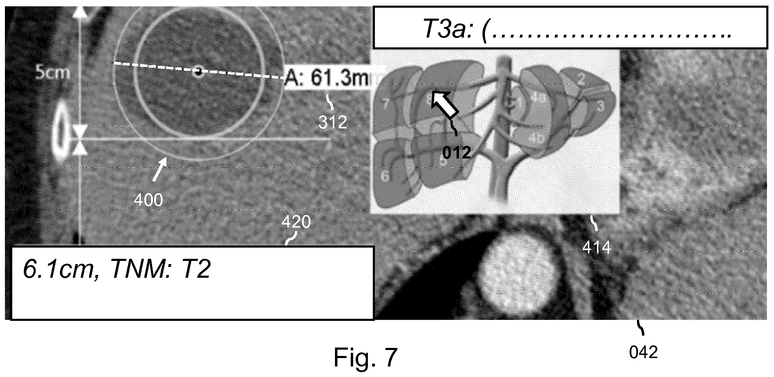

Fig. 6a shows a second overlay 412 being added to the user interface which shows text passages from a medical textbook which describe medical characteristics of the liver lesion stages. In the example of Fig. 6a, the second overlay 412 is shown as a result of the user selecting the text "*T2*, *T3a*" in the first overlay 400, e.g., to indicate a desire to obtain more information. Accordingly, by selecting part of the first overlay 400, the user may be provided with additional information. The first overlay 400 may thus be a selectable overlay, or in general, constitute a selectable graphical element. To provide the user with such additional information, the medical information items in the medical database may be cross-referenced via hyperlinks, and the user interface subsystem may be arranged for enabling the user to interactively browse to another medical information item by selecting a hyperlink in the selectable overlay. Accordingly, the text "*T2, T3a*" in the first overlay 400 may provide or constitute a hyperlink to another medical information item representing the text passages from the medical textbook. The second overlay 412 may show text passages providing an explanatory description of the stages T2 and T3a, such as "*T2: Either a single tumor (any size) that has grown into blood vessels, OR more than one tumor where no tumor is larger than 5 cm (about 2 inches) across"* and "*T3: More than one tumor, with at least one tumor larger than 5cm (about 2 inches) across".* For clearness reasons, however, the above textual explanations have been omitted from Fig. 6a and the following Figs. 6b and 7.

It is noted that the second overlay 412 may also be directly presented to the user after application of the virtual ruler 310 to the medical image 042, thereby constituting another example of the user interface subsystem displaying medical reference information as a selectable graphical element to the user. Moreover, the medical information item which represents the medical reference information may be hyperlinked, thereby enabling the user to browse through the medical textbook by selecting parts of the medical reference information 412. Accordingly, the overlay may progressively disclose relevant textbook information. Moreover, the hyperlinks may be dynamically established based on a current clinical context, such as which body part or organ is displayed. Such contextual information may be obtained from, e.g., metadata of the medical image or a patient record.

Fig. 6a further shows a schematic diagram 414 being added to the user interface. This schematic diagram 414 will be explained in more detail in reference to Fig. 7.

Fig. 6b shows a third overlay 420 being added to the user interface which shows a codified version of the medical reference information. Said codified version has been generated by the reporting subsystem from the medical reference information 412 as well as the measurement 312, for example, by using a macro. The codified version of the medical reference information reads "*A: 6.1cm, TNM: T2*". Accordingly, the report may be generated using the codified version of the medical reference information 420, e.g., by including the abovementioned text passage in the report. Said including may be effected by the user selecting the medical reference information, e.g., by clicking on the second overlay 412. For example, the user may click on the explanatory text of the T2 staging to effect the codified version of the medical reference information being generated for the T2 staging. It is noted that the user interface subsystem may also omit displaying the codified version of the medical reference information 420 to the user. Rather, the reporting subsystem may directly use said codified version 420 in the report, or display said codified version 420 in a different manner.

Fig. 7 shows a fourth overlay of the user interface which shows a schematic drawing 414 of the liver. Compared to Figs. 6a-6b, the fourth overlay 414 is increased in size, e.g., to enable the user select a part of the schematic drawing. The schematic drawing 414 may constitute medical reference information which is displayed to the user as a result of the user selecting the text "T2, T3a" in the first overlay 400, e.g., to indicate a desire to obtain more information. Like the medical reference information in the second overlay 412 of Figs. 6a-6b, the schematic drawing 414 may be hyperlinked in that it allows the user to browse through medical reference information by selecting different parts of the schematic drawing. Accordingly, the user may select an anatomical location in the schematic drawing 414 to request further information on the selected anatomical location. Additionally or alternatively, the selection of the anatomical location may serve as input in generating the codified version of the medical reference information. In particular, the selecting of the anatomical location may constitute input to a macro which then includes a codified version of the anatomical location as part of, or together with the codified version of the medical reference information.

It will be appreciated that the present invention may enable a user to obtain display of medical reference information and to conveniently include a codified version of the medical reference information to a report, namely by selecting the displayed medical reference information. Moreover, the displayed medical reference information may be hyperlinked, thereby enabling the user to refine which medical reference information is used as a basis for generating the codified version of the reference information.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for enabling review of a medical image, comprising:
- an image interface (120) for obtaining the medical image (042);
- a user interface subsystem (140) for enabling a user to apply one or more graphical elements (310) to the medical image during the review of the medical image, thereby establishing application data representing application of the one or more graphical elements to the medical image, wherein the application data comprises data indicating which type of graphical element is applied to the medical image; and
- a reporting subsystem (180) for:
i) accessing a medical database (060) comprising medical information items (062-066) which each represent medical reference information (400-414),
ii) retrieving a medical information item (062) from the medical database based on association data, the association data linking the application of the one or more graphical elements, as represented by the application data, to the medical information item at least based on the data comprised in the application data indicating which type of graphical element applied to the medical image, and
iii) generating a report (182) based on the medical information item.

2. The system (100) according to claim 1, wherein the medical information item (062) is associated with a codified version of the medical reference information (420), and wherein the reporting subsystem (180) is arranged for using the codified version of the medical reference information in generating the report (182).

3. The system (100) according to claim 2, wherein:
- the user interface subsystem (140) is arranged for displaying the medical reference information as a selectable graphical element (400-414) to the user, and
- the reporting subsystem (180) is arranged for using the codified version of the medical reference information (420) in generating the report (182) based on the user selecting the selectable graphical element.

4. The system (100) according to claim 2, wherein the reporting subsystem (180) is arranged for using a macro (080) to generate the codified version of the medical reference information (420) from the medical reference information (400-414).

5. The system (100) according to claim 4, wherein the medical information item (062) comprises the macro (080) or a reference to the macro.

6. The system (100) according to claim 3, wherein the medical information items (062-066) are cross-referenced via hyperlinks, and wherein the user interface subsystem (180) is arranged for enabling the user to interactively browse to another medical information item by selecting a hyperlink in the selectable graphical element (410-414).

7. The system (100) according to claim 3, wherein the selectable graphical element is a selectable overlay (410, 412), and wherein the user interface subsystem is arranged for displaying the medical reference information in the form of text (410, 412) or graphics (414) in the selectable overlay.

8. The system (100) according to claim 1, wherein the one or more graphical elements (310) provide a measurement tool for enabling the user to perform a measurement on the medical image (042) using the user interface subsystem, and wherein the association data is constituted at least in part by measurement data (312) representing the measurement.

9. The system (100) according to claim 8, wherein the reporting subsystem (180) is arranged for using the measurement data (312) in generating the report (182).

10. The system (100) according to claim 1, wherein the association data is constituted at least in part by a plurality of rules each linking a respective one of the one or more graphical elements (310) to a respective one of the medical information items (062-066).

11. The system (100) according to claim 1, wherein the application data represents one or more of the group of:
- a type of the one or more graphical elements (310);
- a position of the one or more graphical elements in the medical image; and
- an image structure (044) marked by the one or more graphical elements.

12. The system (100) according to claim 1, wherein the one or more graphical elements (310) comprise one or more of the group of: a marker, an annotation and a measurement tool (310).

13. Workstation or imaging apparatus comprising the system according to claim 1.

14. A method (200) for enabling review of a medical image, comprising:
- obtaining (210) the medical image;
- enabling (220) a user to apply one or more graphical elements to the medical image during the review of the medical image, thereby establishing application data representing application of the one or more graphical elements to the medical image, wherein the application data comprises data indicating which type of graphical element is applied to the medical image;
- accessing (230) a medical database comprising medical information items which each represent medical reference information;
- retrieving (240) a medical information item from the medical database based on association data, the association data linking the application of the one or more graphical elements, as represented by the application data, to the medical information item at least based on the data comprised in the application data indicating which type of graphical element applied to the medical image, and
- generating (250) a report based on the medical information item.

15. A computer program product (270) comprising instructions that, when executed by a processor system, cause the processor system to perform the method according to claim 14.

## Patentansprüche

1. System (100) zum Ermöglichen einer Überprüfung eines medizinischen Bildes, umfassend:
- eine Bildschnittstelle (120) zum Erhalten des medizinischen Bildes (042);
- ein Anwenderschnittstellensubsystem (140), um einem Anwender zu ermöglichen, ein oder mehrere graphische Elemente (310) auf das medizinische Bild während der Überprüfung des medizinischen Bilds anzuwenden, wodurch Anwendungsdaten hergestellt werden, die Anwendung des einen oder der mehreren grafischen Elemente auf das medizinische Bild darstellen, wobei die Anwendungsdaten Daten umfassen, die anzeigen, welcher Typ von grafischem Element auf das medizinische Bild angewendet wird; und
- ein Meldesubsystem (180) zum:
i) Zugreifen auf eine medizinische Datenbank (060), die medizinische Informationselemente (062-066) umfasst, die jeweils eine medizinische Referenzinformation (400-414) darstellen,
ii) Abrufen eines medizinischen Informationselements (062) aus der medizinischen Datenbank, basierend auf Zuordnungsdaten, wobei die Zuordnungsdaten die Anwendung des einen oder der mehreren grafischen Elemente, wie durch die Anwendungsdaten dargestellt, mit dem medizinischen Informationselement mindestens basierend auf den Daten verknüpfen, die in den Anwendungsdaten umfasst sind, die anzeigen, welcher Typ von grafischem Element auf das medizinische Bild angewendet wird, und
iii) Erstellen eines Berichts (182), basierend auf dem medizinischen Informationselement.

2. System (100) nach Anspruch 1, wobei das medizinische Informationselement (062) mit einer kodierten Version der medizinischen Referenzinformationen (420) verknüpft ist und wobei das Meldesubsystem (180) zur Verwendung der kodierten Version der medizinischen Referenzinformationen beim Erstellen des Berichts (182) angeordnet ist.

3. System (100) nach Anspruch 2, wobei:
- das Anwenderschnittstellensubsystem (140) zum Anzeigen der medizinischen Referenzinformationen als ein auswählbares grafisches Element (400-414) für den Anwender angeordnet ist, und
- das Meldesubsystem (180) zur Verwendung der kodierten Version der medizinischen Referenzinformationen (420) beim Erstellen des Berichts (182) angeordnet ist, basierend auf dem Anwender, der das auswählbare grafische Element auswählt.

4. System (100) nach Anspruch 2, wobei das Meldesubsystem (180) zur Verwendung eines Makros (080) angeordnet ist, um die kodierte Version der medizinischen Referenzinformationen (420) aus den medizinischen Referenzinformationen (400-414) zu erstellen.

5. System (100) nach Anspruch 4, wobei das medizinische Informationselement (062) das Makro (080) oder eine Referenz auf das Makro umfasst.

6. System (100) nach Anspruch 3, wobei die medizinischen Informationselemente (062-066) über Hyperlinks querverwiesen sind und wobei das Anwenderschnittstellensubsystem (180) angeordnet ist, den Anwender zu ermöglichen, interaktiv zu einer anderen medizinischen Informationselementen zu stöbern, indem er einen Hyperlink im auswählbaren grafischen Element (410-414) auswählt.

7. System (100) nach Anspruch 3, wobei das auswählbare grafische Element eine auswählbare Einblendung (410, 412) ist und wobei das Anwenderschnittstellensubsystem angeordnet ist, die medizinischen Referenzinformationen in der Form von Text (410, 412) oder Grafiken (414) in der auswählbaren Einblendung anzuzeigen.

8. System (100) nach Anspruch 1, wobei das eine oder die mehreren grafischen Elemente (310) ein Messwerkzeug bereitstellen, um dem Anwender zu ermöglichen, eine Messung am medizinischen Bild (042) unter Verwendung des Anwenderschnittstellensubsystems auszuführen, und wobei die Verknüpfungsdaten mindestens teilweise von Messdaten (312) begründet werden, die die Messung darstellen.

9. System (100) nach Anspruch 8, wobei das Meldesubsystem (180) angeordnet ist, die Messdaten (312) beim Erstellen des Berichts (182) zu verwenden.

10. System (100) nach Anspruch 1, wobei die Verknüpfungsdaten mindestens teilweise von einer Vielzahl von Regeln begründet werden, die jeweils ein jeweiliges des einen oder der mehreren grafischen Elemente (310) mit einem jeweiligen der medizinischen Informationselemente (062-066) verbinden.

11. System (100) nach Anspruch 1, wobei die Anwendungsdaten eines oder mehreres der Gruppe darstellen von:
- einem Typ des einen oder der mehreren grafischen Elemente (310);
- einer Position des einen oder der mehreren grafischen Elemente im medizinischen Bild; und
- einer Bildstruktur (044), die von dem einen oder den mehreren grafischen Elementen markiert ist.

12. System (100) nach Anspruch 1, wobei das eine oder die mehreren grafischen Elemente (310) eines oder mehrere der Gruppe umfassen von: einer Markierung, einer Beschriftung und einem Messwerkzeug (310).

13. Arbeitsstation oder Bildgebungseinrichtung, umfassend das System nach Anspruch 1.

14. Verfahren (200) zum Ermöglichen einer Überprüfung eines medizinischen Bilds, umfassend:
- Erhalten (210) des medizinischen Bilds:
- Ermöglichen (220), dass ein Anwender ein oder mehrere grafische Elemente auf das medizinische Bild während der Überprüfung des medizinischen Bilds anwendet, wodurch Anwendungsdaten hergestellt werden, die Anwendung des einen oder der mehreren grafischen Elemente auf das medizinische Bild darstellen, wobei die Anwendungsdaten Daten umfassen, die anzeigen, welcher Typ von grafischem Element auf das medizinische Bild angewendet wird;
- Zugreifen (230) auf eine medizinische Datenbank, die medizinische Informationselemente umfasst, die medizinische Referenzinformationen darstellen;
- Abrufen (240) eines medizinischen Informationselements aus der medizinischen Datenbank, basierend auf Verknüpfungsdaten, wobei die Verknüpfungsdaten die Anwendung des einen oder der mehreren grafischen Elemente, wie von den Anwendungsdaten dargestellt, mit dem medizinischen Informationselement mindestens basierend auf den Daten verbinden, die in den Anwendungsdaten umfasst sind, die anzeigen, welcher Typ von grafischem Element auf das medizinische Bild angewendet wird, und
- Erstellen (250) eines Berichts, basierend auf dem medizinischen Informationselement.

15. Computerprogrammprodukt (270), umfassen Befehle, die, wenn von einem Prozessorsystem ausgeführt, das Prozessorsystem veranlassen, das Verfahren nach Anspruch 14 durchzuführen.

## Revendications

1. Système (100) pour permettre la révision d'une image médicale, comprenant :
- une interface d'image (120) pour obtenir l'image médicale (042) ;
- un sous-système d'interface utilisateur (140) pour permettre à un utilisateur d'appliquer un ou plusieurs éléments graphiques (310) à l'image médicale au cours de la révision de l'image médicale, établissant de la sorte des données d'application représentant l'application des un ou plusieurs éléments graphiques à l'image médicale, dans lequel les données d'application comprennent des données indiquant quel type d'élément graphique est appliqué à l'image médicale ; et
- un sous-système d'établissement de rapports (180) pour :
i) accéder à une base de données médicale (060) comprenant des articles d'information médicaux (062-066) qui représentent chacun des informations de référence médicales (400-414),
ii) récupérer un article d'information médical (062) de la base de données médicales sur la base de données d'association, les données d'association liant l'application des un ou plusieurs éléments graphiques, tel que représentés par les données d'application, à l'article d'information médicale au moins sur la base des données comprises dans les données d'application indiquant quel type d'élément graphique est appliqué à l'image médicale, et
iii) générer un rapport (182) sur la base de l'article d'information médical.

2. Système (100) selon la revendication 1, dans lequel l'article d'information médical (062) est associé à une version codifiée des informations de référence médicales (420) et dans lequel le sous-système d'établissement de rapport (180) est agencé pour utiliser la version codifiée des informations de référence médicales en générant le rapport (182).

3. Système (100) selon la revendication 2, dans lequel :
- le sous-système d'interface utilisateur (140) est agencé pour afficher les informations de référence médicales en tant qu'élément graphique sélectionnable (400, 414) auprès de l'utilisateur et
- le sous-système d'établissement de rapports (180) est agencé pour utiliser la version codifiée des informations de référence médicales (420) en générant le rapport (182) sur la base de la sélection par l'utilisateur de l'élément graphique sélectionnable.

4. Système (100) selon la revendication 2, dans lequel le sous-système d'établissement de rapports (180) est agencé pour utiliser une macro (080) afin de générer la version codifiée des informations de référence médicales (420) venant des informations de référence médicales (400-414).

5. Système (100) selon la revendication 4, dans lequel l'article d'information médical (062) comprend la macro (080) ou une référence à la macro.

6. Système (100) selon la revendication 3, dans lequel les articles d'information médicaux (062-066) sont renvoyés via des hyperliens et dans lequel le sous-système d'interface utilisateur (180) est agencé pour permettre à l'utilisateur d'explorer de manière interactive un autre article d'information médical en sélectionnant un hyperlien dans l'élément graphique sélectionnable (410-414).

7. Système (100) selon la revendication 3, dans lequel l'élément graphique sélectionnable est un recouvrement sélectionnable (410, 412) et dans lequel le sous-système d'interface utilisateur est agencé pour afficher les informations de référence médicales sous la forme d'un texte (410, 412) ou d'un graphique (414) dans le recouvrement sélectionnable.

8. Système (100) selon la revendication 1, dans lequel les un ou plusieurs éléments graphiques (310) fournissent un outil de mesure pour permettre à l'utilisateur d'effectuer une mesure sur l'image médicale (042) en utilisant le sous-système d'interface utilisateur et dans lequel les données d'association sont constituées au moins en partie de données de mesure (312) représentant la mesure.

9. Système (100) selon la revendication 8, dans lequel le sous-système d'établissement de rapports (180) est agencé pour utiliser les données de mesure (312) en générant le rapport (182).

10. Système (100) selon la revendication 1, dans lequel les données d'association sont constituées au moins en partie d'une pluralité de règles liant chacune l'un respectif des un ou plusieurs éléments graphiques (310) à l'un respectif des articles d'information médicaux (062-066).

11. Système (100) selon la revendication 1, dans lequel les données d'application représentent un ou plusieurs du groupe des suivantes :
- un type des un ou plusieurs éléments graphiques (310) ;
- une position des un ou plusieurs éléments graphiques dans l'image médicale ; et
- une structure d'image (044) marquée par les un ou plusieurs éléments graphiques.

12. Système (100) selon la revendication 1, dans lequel les un ou plusieurs éléments graphiques (310) comprennent un ou plusieurs du groupe parmi un marqueur, une annotation et un outil de mesure (310).

13. Station de travail ou appareil d'imagerie comprenant le système selon la revendication 1.

14. Procédé (200) pour permettre la révision d'une image médicale, comprenant :
- l'obtention (210) de l'image médicale ;
- la capacité (220) pour un utilisateur d'appliquer un ou plusieurs éléments graphiques à l'image médicale au cours de la révision de l'image médicale, établissant de la sorte des données d'application représentant l'application des un ou plusieurs éléments graphiques à l'image médicale, dans lequel les données d'application comprennent des données indiquant quel type d'élément graphique est appliqué à l'image médicale ;
- l'accès (230) à une base de données médicales comprenant des articles d'information médicaux qui représentent chacun des informations de référence médicales ;
- la récupération (240) d'un article d'information médical dans la base de données médicales sur la base de données d'association, les données d'association liant l'application des un ou plusieurs éléments graphiques, tels que représentés par les données d'application, à l'article d'information médical au moins sur la base des données comprises dans les données d'application indiquant quel type d'élément graphique est appliqué à l'image médicale, et
- la génération (250) d'un rapport sur la base de l'article d'information médical.

15. Produit de programme d'ordinateur (270) comprenant des instructions qui, lorsqu'elles sont exécutées par un système de processeur, amènent le système de processeur à effectuer le procédé selon la revendication 14.
